# EUROPEAN PATENT APPLICATION

(11) **EP 2 870 964 A2**
(43) Date of publication of application: **13.05.2015**
(21) Application number: 13813180.0
(22) Date of filing: 03.07.2013
(51) Int. Cl.: A61K 31/16, A61K 31/165, A61P 29/00

(54) **COMPOSITION FOR PREVENTING OR TREATING COLITIS COMPRISING S-ALLYL-L-CYSTEINE AS ACTIVE INGREDIENT, AND MEDICAL PREPARATION COMPRISING SAME**

(30) Priority: 03.07.2012 KR 20120072080
(71) Applicant: Pharmaking Co., Ltd., Eumseong-gun, Chungcheongbuk-do 369-852 (KR)
(72) Inventor: KIM, Soon Bae, Seongnam-si Gyeonggi-do 463-802 (KR); KIM, Gwang Soon, Seongnam-si Gyeonggi-do 463-848 (KR); KIM, Wan Bae, Seoul 110-797 (KR); KWAK, Wie Jong, Seoul 137-764 (KR); JEON, Sun Duck, Seoul 156-879 (KR); YOON, Hyung Young, Seoul 156-030 (KR); HAHM, Ki Baik, Seongnam-si Gyeonggi-do 463-728 (KR); KIM, Eun Hee, Bucheon-si Gyeonggi-do 420-031 (KR)
(74) Representative: Gulde & Partner
(86) International application number: PCT/KR2013/005922
(87) International publication number: WO 2014/007549

(57) **Abstract**

The present invention relates to a composition for preventing or treating colitis. According to one embodiment of the present invention, the composition for preventing or treating colitis comprises S-allyl-L-cysteine, a pharmaceutically acceptable salt thereof, or a solvate or hydrate of the same as an active ingredient, and exhibits an anti-inflammatory action in colitis. The composition for preventing or treating colitis has outstanding effects in preventing and alleviating colitis due to the effect of the anti-inflammatory action and the effect of an antioxidant action of S-allyl-L-cysteine, while at the same time S-allyl-L-cysteine is not accompanied by side-effects, therefore, has a wide scope of use against colitis.

## Description

### BACKGROUND

### Technical Field

The present invention relates to a composition for preventing or treating colitis. More particularly, the present invention relates to a composition for the prevention or treatment of colitis, comprising S-allyl-L-cysteine as an active ingredient, and a use thereof.

### Background Art

Colitis is largely classified into two types: infectious colitis, such as amoebic dysentery, giardiasis, intestinal tuberculosis, mycosis, etc., and non-specific colitis such as Crohn's disease, ulcerative colitis, etc.

*Inter alia,* ulcerative colitis and Crohn's disease are problematic and difficult to treat because unlike infectious colitis their causes and etiology are unknown. In the past, most cases of chronic colitis were attributed to intestinal tuberculosis, but cases of ulcerative colitis and Crohn's disease have gradually increased with the Westernization of life environments.

Ulcerative colitis is characterized by inflammation generated in the mucous membrane and the submucosal tissue, but does not extend to the muscle layer and the serous membrane. This disease is prevalent in the anus and the rectum, with the accompaniment of symptoms including painful abdominal cramping with bowel movements, diarrhea mixed with blood, release of excrement with purulent matter or viscous liquid, etc. When entering the chronic phase, ulcerative colitis may develop into colorectal cancer.

So far, no modalities have been known to completely cure ulcerative colitis. Current internal therapy for ulcerative colitis includes inflammation relief, tissue injury healing, and mitigation of symptoms such as diarrhea, hematochezia, abdominal pain, etc. For internal therapy, it is preferentially necessary to accurately determine the site, coverage, and degree of ulcerative colitis. Therapeutic effects are evaluated on the basis of pre-therapeutic conditions.

There are no direct known causes for ulcerative colitis, but some knowledge about the onset and development of ulcerative colitis has been accumulated to a degree. Based on this knowledge, various medicines have been developed and applied. Among them are anti-inflammatory agents such as sulfasalazine and mesalazine, adrenal cortex hormones such as prednisone, prednisolone, hydrocortisone, ENTOCORT, and budesonide, immunosuppressants such as IMMURAN, 6-MP, cyclosporine and methotrexate, antibiotic agents such as metronidazole and CIPROBAY, internal cavity modifiers such as short-chain fatty acids and glutamine, antioxidants such as allopurinol and dimethyl sulfoxide, local anesthetics such as lidocaine, and other medicines including nicotine, heparin, ketotifen, thalidomide, colony stimulating factor, and epidermal growth factors.

Crohn's disease is a type of inflammatory bowel disease that may affect any part of the gastrointestinal tract from the mouth to the anus, but frequently affects the terminal large intestine adjacent to the cecum, with the accompaniment of gastrointestinal symptoms including diarrhea, weight loss, lower abdominal pain, fever, and proctorrhagia. Also, other complications may occur outside the gastrointestinal tract and include anemia, nutrient deficiency, musculoskeletal deformity, renal dysfunction, and eye inflammation. Often, a complication, such as darmstenose, intestinal abscess, or intestinal fistula, may occur and those with the disease are at greater risk of bowel cancer. Crohn's disease results in a chronic inflammatory disorder with a high recurrence rate and is apt to develop granuloma deeply within the walls of the intestine. Recently, many cases of Crohn's disease are being detected in young people in their twenties and thirties, but its cause remains unknown. Crohn's disease should be treated in a modality different from that applied to other bowel diseases such as infectious bowel disease, ischemic bowel disease, ulcerative colitis, intestinal tuberculosis, etc. After accurate diagnosis with endoscopic biopsy and radiographic imaging, Crohn's disease is treated with conservative therapy such as infusion in combination with pharmacotherapy. Drug therapy is mainly used, including steroids for severe symptoms, and sulfasalazine or mesalazine for mild symptoms. To prevent complications or to reduce recurrence, those with Crohn's disease must take drugs for their entire lives. When complications are not controlled with drugs, a surgical operation may be necessary, but even so, such a procedure cannot completely cure Crohn's disease. Even after an operation, Crohn's disease may recur.

For use as an anti-inflammatory agent in treating ulcerative colitis and Crohn's disease, sulfasalazine is a drug in which the sulfa-based antibiotic sulfapyridine is associated with 5-aminosalicylic acid (5-ASA), 5-ASA being similar to aspirin. In the body, intestinal bacteria metabolize sulfasalazine into 5-ASA and sulfapyridine. Of the metabolites, the free 5-ASA exhibits anti-inflammatory activity. Sulfasalazine is useful for the therapy of ulcerative colitis as well as Crohn's and Behcet's disease, which both affect the large intestine. The drug is prescribed to prevent ulcerative diseases from recurring after remission. However, sulfasalazine's effect on preventing the recurrence of Crohn's disease has yet to be proven. Side effects of sulfasalazine often include skin rash, nausea, abdominal pain, and hepatic dysfunction, and in rare cases myelosuppression.

Since side effects of sulfasalazine are, for the most part, attributed to the sulfapyridine molecule, mesalazine (also known as metalamine), which is a derivative of sulfasalazine free of sulfapyridine, was developed. When ingested, 5-ASA is absorbed into parts of the small intestine unaffected by disease, but cannot reach the affected site(s). To reach affected regions, 5-ASA is specially modified in various manners. Mesalazine is similar in effect to sulfasalazine, but with somewhat fewer side effects. Mesalazine may be administered orally or formulated into suppositories or enemas. Suppositories or enemas may be effectively applied to the inflammation of the rectum and the left colon, respectively, or may be used with the aim of maintaining remission. Oral mesalamine is effective not only for the therapy of active ulcerative colitis, Crohn's disease, and Behcet's disease, but also in maintaining the remission of these diseases.

To deliver 5-ASA to the terminal small intestine or the large intestine, various mesalazine formulations have been developed and are now commercially available. Examples include dipentum (olsalazine) in which two 5-ASA molecules are bonded to each other; COLAZIDE and ipsalazide, which employ non-toxic molecules instead of sulfapyridine; pentasa and azalan in which 5-ASA is adsorbed to cellulose or resin so that 5-ASA is slowly released; and asacol, rowasa, and salofalk (claversal, mesasal), which are configured to release 5-ASA when intestinal acidity (pH) turns alkaline. Sulfasalazine and dipentum are effective for the therapy of colon inflammation while asacol, salofalk and pentasa may be useful in the treatment of inflammation even in the small intestine.

Although widely used to treat inflammatory bowel diseases such as ulcerative colitis or Crohn's disease, mesalazine formulations cause side effects including hypersensitivity reactions such as allergic skin rashes, hyperpyrexia, bronchospasm, and lupus erythematosus, vomiting, nausea, and headache.

Hence, active research has been directed toward novel compositions that are effective in preventing or ameliorating inflammatory bowel diseases, but without the accompaniment of side effects.

An art related with the present invention may be found in non-patent document 1 below. It discloses that Sulfasalazine and steroids have a treating effect in Carrageenans-induced inflammatory animal models, and, steroids and immunosuppressants inhibits the generation of cytokines in immune cells such as T cells. However, sulfasalazine and steroid formulations cause not only the above-mentioned side effects, but steroid formulations rapidly increase body weight. For these reasons, severe limitations are imparted to the use of the formulations.

(Non-Patent Document 1) Cronstein BN, Montesinos MC and Weissman G, Salitylate and sulfasalazine, but not glucocorticoid, inhibit leukocyte accumulation by an adenosinedependent mechanism that is independent of inhibition of prostaglandin synthesis and p105 NF-κB, Proc. Natl. Acad. Sci. USA, May 25, 1999, 96(11): 6377-6381

### SUMMARY

It is an object of the present invention to provide a pharmaceutical composition for the prevention or treatment of colitis that can bring about excellent improvement in treating and preventing colitis, without side effects.

It is another object of the present invention to provide a use of the pharmaceutical composition in the prevention or treatment of colitis.

In order to accomplish the above objects, an aspect of the present invention provides a pharmaceutical composition for the prevention or treatment of colitis, the composition comprising S-allyl-L-cysteine, a pharmaceutically acceptable salt thereof, or a solvate or hydrate thereof as an active ingredient, and exhibits anti-inflammatory activity against colitis.

The composition for the prevention or treatment of colitis comprise contain S-allyl-L-cysteine, a pharmaceutically acceptable salt thereof, or a solvate or hydrate thereof as an active ingredient, and may exhibits anti-inflammatory and anti-oxidant activity.

The composition for the prevention or treatment of colitis may contain S-allyl-L-cysteine in an amount of 5 to 99.9 wt %.

In the composition for the prevention or treatment of colitis, S-allyl-L-cysteine may be obtained by isolation and purification from the plant Allium genus, by synthesis, or by fermentation.

The composition for the prevention or treatment of colitis may further comprise an anti-inflammatory agent or an anti-oxidant agent.

In the composition for the prevention or treatment of colitis, the anti-inflammatory agent may be selected from the group consisting of ibuprofen, ketoprofen, flurbiprofen, feno-profen, naproxen, piroxicam, tenoxicam, isoxicam, melo-xicam, indomethacin, aceclofenac, diclofenac, and a combination thereof, and the anti-oxidant agent may be selected from the group consisting of vitamin A, vitamin C, vitamin E, carotenoid, zinc, copper, iron, manganese, lutein, zeaxanthin, selenium, glutathione (GSH), lycopene, and a combination thereof.

Contemplated in accordance with another aspect of the present invention is a medicinal formulation, comprising the composition for the prevention or treatment of colitis, the medicinal formulation being selected from the group consisting of an oral dosage form, a mucosal application, an injection, an inhaler, and an external application.

Below, a detailed description will be given of the present invention.

In accordance with an aspect thereof, the present invention addresses a pharmaceutical composition for the prevention or treatment of colitis, the composition comprising S-allyl-L-cysteine, a pharmaceutically acceptable salt thereof, or a solvate or hydrate thereof as an active ingredient, and exhibits anti-inflammatory activity against colitis.

S-allyl-L-cysteine is a natural constituent of mature garlic, and is reported to exhibit various pharmaceutical efficacies including a suppressive effect on arteriosclerosis due to its antioxidant activity, and an inhibitory effect on some cancer cell lines (Proceedings of the American Association for Cancer Research, 30, p181, 1989).

As will be elucidated in detail in the following Example section, 8-oxo-deoxyguanosine, the composition comprising S-allyl-L-cysteine as an active ingredient, and preventive and curative of colitis, established its preventive effect on colitis thanks to its anti-inflammatory activity in dextran sodium sulfate (DSS)-induced colitis mouse models.

In addition, because S-allyl-L-cysteine or a pharmaceutically or cytologically acceptable salt thereof may be in the form of solvates or hydrates, the composition of the present invention may utilize a solvate or hydrate of S-allyl-L-cysteine or the pharmaceutically acceptable salt as an active ingredient.

As mentioned above, the composition for the prevention or treatment of colitis may comprise a pharmaceutically or cytologically acceptable salt of S-allyl-L-cysteine as an active ingredient. An acid addition salt or a quaternary ammonium salt may also be used. Examples of acid addition salts include inorganic acid salts such as hydrochloride, hydrobromide, hydroiodide, sulfate, phosphate, etc., and organic acid salts such as oxalate, maleate, fumarate, lactate, maleate, succinate, tartrate, benzoate, methanesulfonate, etc., but are not limited thereto. Examples of quaternary ammonium salts include but are not limited to lower alkyl halogenides such as methyl iodide, methyl bromide, ethyl iodide, ethyl bromide, and the like; lower alkyl sulfonates such as methylmethane sulfonate, ethylmethane sulfonate, and the like; and lower alkyl aryl sulfonate such as methyl-p-toluene sulfonate.

In addition, as will be explained in the following Example section, S-allyl-L-cysteine was also found to exert excellent antioxidant activity on colitis.

Containing as the active ingredient S-allyl-L-cysteine having both anti-inflammatory activity and anti-oxidant activity against colitis, the composition of the present invention can exhibit excellent preventive and therapeutic effects on colitis even when not using two or more agents in combination.

Sulfasalazine, conventionally used for the treatment of colitis, causes side effects often including skin rashes, nausea, abdominal pain, and hepatic dysfunction, and in rare cases myelosuppression. Further, the conventional agent has difficulty in maintaining remission. In contrast, S-allyl-L-cysteine, which is used as an active ingredient in the pharmaceutical composition for the prevention or treatment of colitis according to the present invention, produces neither the side effects caused by sulfasalazine, nor requires the use of a steroidal agent in combination, so that the composition can find a broad range of applications in preventing and treating colitis.

In one embodiment, the composition for the prevention or treatment of colitis may comprise S-allyl-L-cysteine in an amount of 5 to 99.9 weight %.

Given such an amount of S-allyl-L-cysteine, the composition not only exhibits both anti-inflammatory and anti-oxidant activities simultaneously to thus effectively treat colitis, but does not produce side effects in contrast to general treating agents such as sulfasalazine.

The colitis treatable in the present invention includes infectious colitis and inflammatory bowel diseases. The inflammatory bowel diseases include but are not limited to idiopathic, chronic, and non-specific colitis such as ulcerative colitis and Crohn's disease, as well as typical inflammatory conditions of the colon and small intestine.

As an active ingredient in the composition for the prevention or treatment of colitis, S-allyl-L-cysteine may be obtained by isolation and purification from the plant Allium genus, by synthesis, or by fermentation. For example, S-allyl-L-cysteine may be prepared from Allium genus, such as garlic, elephant garlic, onion, scallion, etc., using the method disclosed in paragraph [0031] of EP 0429080A1. Alternatively, S-allyl-L-cysteine may be synthesized or prepared using a technique known in the art, such as fermentation.

S-allyl-L-cysteine, a pharmaceutically or cytologically acceptable salt thereof, or a solvate or hydrate thereof, all serving as active ingredients of the composition of the present invention, may be administered in their natural states to patients, or as a composition containing one or two active ingredients, or as a complex formulation with other anti-inflammatory agents or anti-oxidant agents.

The anti-inflammatory agent useful in the present invention may be selected from the group consisting of ibuprofen, ketoprofen, flurbiprofen, feno-profen, naproxen, piroxicam, tenoxicam, isoxicam, melo-xicam, indomethacin, aceclofenac, diclofenac, and a combination thereof. The anti-oxidant may be selected from the group consisting of vitamin A, vitamin C, vitamin E, carotenoid, zinc, copper, iron, manganese, lutein, zeaxanthin, selenium, glutathione (GSH), lycopene, and a combination thereof.

According to one embodiment of the present invention, the composition for the prevention or treatment of colitis may be prepared into a formulation selected from the group consisting of an oral dosage form, a mucosal application, an injection, an inhaler, and an external application.

Examples of the oral agent include hard and soft capsules, tablets, suspensions, syrups, powders, sustained-release agents, enteric agents, granules, oleo saccharum, fine granules, pills, extracts, liquids, aromatic water, emulsions, syrups, elixirs, fluid extracts, precipitants, tinctures, spirits, and medicinal emulsions, but are not limited thereto. The mucosal application may be exemplified by, but not limited to, troche, buccal tablets, sublingual tablets, suppositories, and nasal sprays. Examples of the injection include subcutaneous injections, intramuscular injections, intravenous injections, and intravitreal drug implants, but are not limited thereto. Transnasal agents, eye drops, ear drops, ophthalmic ointments, pastes, cataplasma, liniments, lotions, coatings, sprays, and external use liquids fall within the scope of the external application.

In addition to one or more active ingredients, the composition of the present invention may further comprise one or more inert, pharmaceutically acceptable vehicles, for example, excipients such as starch, lactose, carboxymethyl cellulose, kaolin, etc., binders such as water, gelatin, alcohol, glucose, Arabic rubber, tragacanth, etc., disintegrants such as starch, dextrin, sodium alginate, etc., lubricants such as talc, stearic acid, magnesium stearate, fluid paraffin, etc., and additives such as solubilizers.

When the composition of the present invention is used as a food composition, the amount of the active ingredient may be determined according to the purpose of the composition (e.g., prevention or treatment of diseases, health aids, etc.). Generally, S-allyl-L-cysteine may be added in an amount of 0.0001 to 90 wt %, and preferably in an amount of 0.1 to 50 wt % when used to prepare foods or drinks. If the foods or drinks are designed for long-term ingestion to provide health control and sanitation, the amount may be further decreased, but adverse events are observed when used in greater amounts because the active ingredient is not toxic to the body. There is no particular limitation on the kind of health food to which the composition of the present invention can be added. Examples of such health foods include meats, sausages, breads, chocolates, candies, snacks, confectionery, pizzas, ramen noodles, other noodles, gums, dairy products such as ice-cream, various soups, beverages, teas, drinks, alcoholic beverages, and vitamin complexes.

### Advantageous Effects

Containing as the active ingredient S-allyl-L-cysteine having both anti-inflammatory activity and anti-oxidant activity against colitis, the composition of the present invention can exhibit excellent preventive and therapeutic effects on colitis, and does not produce side effects. Hence, the composition can find a broad range of applications in the prophylaxis and therapy of colitis.

Also, a use of the pharmaceutical composition of the present invention in preventing or treating colitis is provided in accordance with an embodiment of the present invention.

### BRIEF DESCRIPTION OF DRAWINGS

FIG. 1 is a graph of body weights of mice exposed to dextran sodium sulfate (DSS).
FIG. 2 is a photographic image of colons from mice treated with compositions of Example, Comparative Example, and Control Example after autopsy.
FIG. 3 is graph showing disease activity index (DAI) scores of mice by group.
FIG. 4 is a graph showing colon lengths of mice by group.
FIGS. 5 and 6 show pathologic data of mice treated with compositions of Example, Comparative Example, and Control Example in images (FIG. 5) and in a graph (FIG. 6), as analyzed by hematoxylin & eosin staining (H&E stain).
FIGS. 7 to 10 are graphs respectively showing expression levels of inflammatory cytokines IL-1β, IL-6, TNF-α, and IL-12 in mice treated with compositions of Example, Comparative Example, and Control Example.
FIGS. 11 and 12 are graphs showing the anti-oxidant activity of the compositions of Example, Comparative Example, and Control Example, as measured for MDA (FIG. 11) and antioxidant concentration (FIG. 12).
FIG. 13 shows immunofluorescent images illustrating suppressive effects of the compositions of Example, Comparative Example, and Control Example on COX-2 expression.

### DETAILED DESCRIPTION

A better understanding of the present invention may be obtained through the following examples which are set forth to illustrate, but are not to be construed as limiting the present invention.

### [PREPARATION EXAMPLE: Preparation of Compositions Used in Example, Comparative Example, and Control Example]

Compositions were prepared as shown in Table 1 below. For comparison in terms of therapeutic effect and side effect, rebamipide, a conventional therapeutic agent for colitis, was employed as a control.

**TABLE 1**

| | Example 1 | Example 2 | Example 3 | Example 4 | Comparative Example | Control |
|---|---|---|---|---|---|---|
| S-allyl-L-cysteine | 100 | 50 | 25 | 12.5 | 0 | 0 |
| Drinking Water | 0 | 50 | 75 | 87.5 | 100 | 0 |
| Rebamipide | 0 | 0 | 0 | 0 | 0 | 100 |

| | | | | | | |
|---|---|---|---|---|---|---|
| - S-allyl-L-cysteine: from Hana Pharm Co. Ltd., Korea - Rebamipide: from Ostuka Pharmaceutical | | | | | | |

### [EXPERIMENTAL EXAMPLE 1: Induction of Inflammatory Bowel Disease by Dextran Sodium Sulfate]

### (1) Preparation of experimental animal

In this experiment, C57BL6 mice were employed for the following reasons. The experimental animals are widely used throughout the world and are evaluated as being suitable for validity assays. In addition, because a great amount of fundamental test data on various disease models of the animals has been accumulated, the data can be utilized in analyzing and assessing test results. When purchased from OrientBio Inc. the experimental animals weighed 18 g ± 20%. They were bred under a light intensity of 150∼300 Lux in the controlled condition of 12:12-h light-dark cycles, with at least 10 ventilation rounds per hour.

When starting the experiment, the mice in each group weighed, on average: 20.8±1.4 g for G1; 21.0±0.5 g for G2; 20.9±1.5 g for G3; 20.2±0.9 g for G4; 20.7±1.1 g for G5; 20.5±0.9 g for G6; and 20.7±0.7 g for G7, with no significant difference between groups.

### (2) Colitis induction

For use in testing therapeutic effects of the agents of Examples, Comparative Examples, and Control Examples, the mice (G1 to G7) were fed with a 2.5 wt % aqueous dextran sodium sulfate (DSS) solution to induce inflammatory bowel disease therein.

On the day of induction of inflammatory bowel disease, no animals were dead or in a critical condition.

### [EXPERIMENTAL EXAMPLE 2: Therapeutic Effect of S-allyl-L-cysteine on Colitis]

### Experimental Example 2-1. Treatment with Compositions of Example 1, Comparative Example, and Control Example

The mice were pre-treated with compositions of Example 1, Comparative Example, and Control Example as shown in Table 2, and a couple of weeks after the starting of the pretreatment, DSS was used to induce colitis in the animal models.

**TABLE 2**

| Test Group | Disease Inducer | Test Composition | Dose(g/kg) | No. of Animals |
|---|---|---|---|---|
| G1 (Normal) | - | - | - | 10 |
| G2 | DSS 2.5 wt% | C. Example | 50mg/kg | 10 |
| G3 | DSS 2.5 wt % | Ex. 1 | 12.5mg/kg | 10 |
| G4 | DSS 2.5 wt % | Ex. 1 | 25mg/kg | 10 |
| G5 | DSS 2.5 wt % | Ex. 1 | 50mg/kg | 10 |
| G6 | DSS 2.5 wt % | Ex. 1 | 100mg/kg | 10 |
| G7 | DSS 2.5 wt % | Control | 50mg/kg | 10 |

### Experimental Example 2-2. Fundamental Observation Result

The animals were measured to have body weights of: G1: 23.4±1.5, G2: 23.1±1.2, G3: 22.1±2.4, G4: 22.8±0.8, G5: 22.9±1.1, G6: 22.2±1.3, and G7: 23.2±1.2 at the time of DSS treatment, and weighed: G1: 24.2±1.7, G2: 21.9±1.2, G3: 20.3±2.2, G4: 20.5±0.9, G5: 21.4±0.8, G6: 21.6±0.7, and G7: 21.1±0.9 at the time of autopsy.

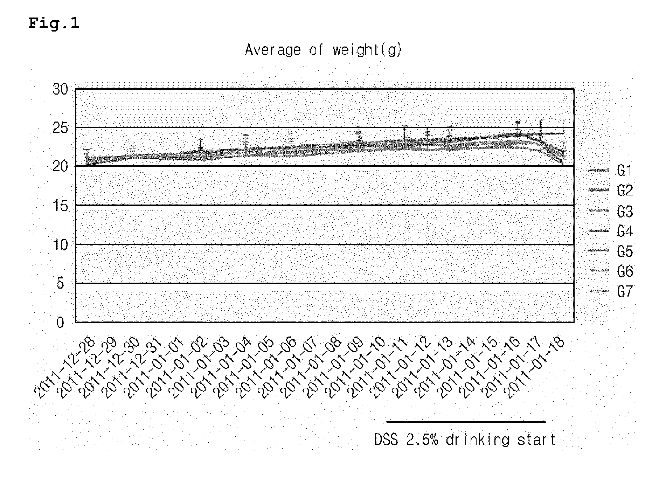

Weights of each group by day are summarized in Tables 3 and FIG. 1. Weights are expressed as mean ± standard deviation.

**TABLE 3**

| | G1 (g) | G2 (g) | G3 (g) | G4 (g) | G5 (g) | G6 (g) | G7 (g) |
|---|---|---|---|---|---|---|---|
| 2011.12.28 | 20.8±1.4 | 21.0±0.5 | 20.9±1.5 | 20.2±0.9 | 20.7±1.1 | 20.5±0.9 | 20.7±0.7 |
| 2011.12.29 | | | | | | | |
| 2011.12.30 | | | | | | | |
| 2011.12.31 | | | | | | | |
| 2012.01.01 | | | | | | | |
| 2012.01.02 | | | | | | | |
| 2012.01.03 | | | | | | | |
| 2012.01.04 | | | | | | | |
| 2012.01.05 | | | | | | | |
| 2012.01.06 | | | | | | | |
| 2012.01.07 | | | | | | | |
| 2012.01.08 | | | | | | | |
| 2012.01.09 | | | | | | | |
| 2012.01.10 | | | | | | | |
| 2012.01.11 | | | | | | | |
| 2012.01.12 | 23.4±1.5 | 23.1±1.2 | 22.1±2.4 | 22.8±0.8 | 22.9±1.1 | 22.2±1.3 | 23.2±1.2 |
| 2012.01.13 | | | | | | | |
| 2012.01.14 | | | | | | | |
| 2012.01.15 | | | | | | | |
| 2012.01.16 | | | | | | | |
| 2012.01.17 | | | | | | | |
| 2012.01.18 | 24.2±1.7 | 21.9±1.2 | 20.3±2.2 | 20.5±0.9 | 21.4±0.8 | 21.6±0.7 | 21.1±0.9 |

From January 12, 2012, the mice were allowed to drink an aqueous DSS 2.5 wt % solution for 7 days. DSS-induced diarrhea or hematochezia caused mice in groups G2 to G7 to lose weight, as compared at the point of DSS treatment.

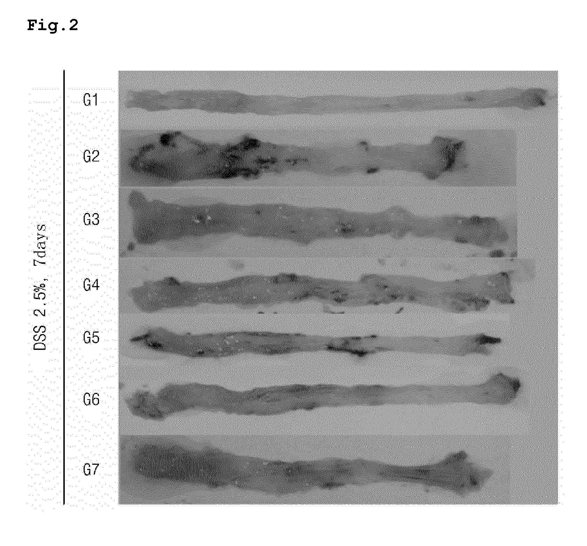

After 7 days of DSS drinking, the mice were sacrificed in an ether anesthetized condition, and the colon was excised. The colons from G2 to G7 were observed to have inflammation as a result of DSS-induced diarrhea or hematochezia, as shown in FIG. 2.

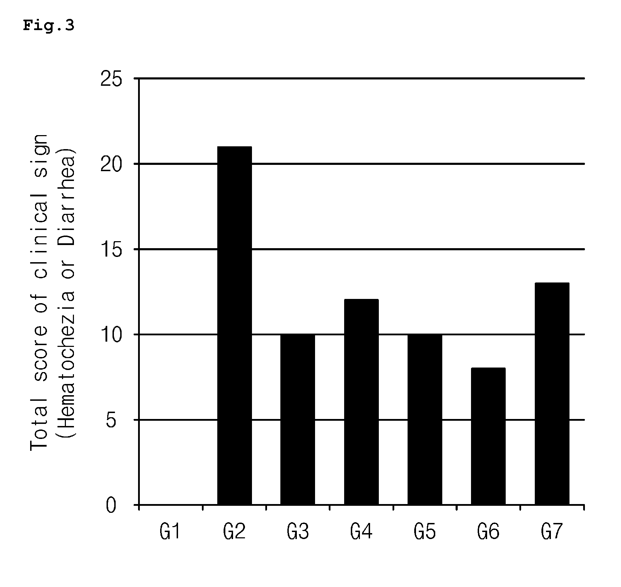

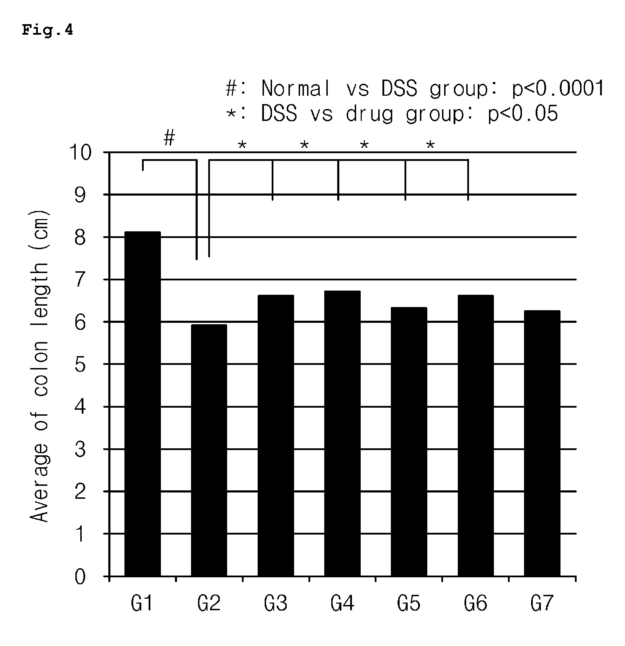

At the end of the test, Disease Activity Index (DAI) Scores were made in consideration of the degree of hematochezia and diarrhea, and are depicted in FIG. 3. In addition, the overall colon length from the cecum to the end of the colon was measured, and the result is shown in FIG. 4. In the graph, # represents significance (p<0.0001) compared to the normal (G1), and * represents significance (p<0.05), compared to the colitis animal model.

**TABLE 4**

| Score of Disease Activity Index, and Criteria | | |
|---|---|---|
| Score | Stool consistency | Occult or Gross rectal bleeding |
| 0 | Normal stool | Negative |
| 1 | Loose stool | Negative |
| 2 | Loose stool | Positive |
| 3 | Diarrhea | Positive |
| 4 | Diarrhea | Gross bleeding |

As understood from the data of FIG. 3, the inflammatory bowel disease induced by DSS caused a clinical sign change such as diarrhea and hematochezia due to the inflammation in the colon mucosa, increasing the DAI score whereas treatment with the composition of the present invention relieved the inflammation of the colon mucosa to mitigate diarrhea and hematochezia, thus allowing mice in G3 to G6 to score low in DAI. In addition, as shown in FIG. 4, DSS-induced colitis made the colon shorter, compared to the normal group. Treatment of the composition of the present invention recovered the colon length, as pointed out in G3 to G6 compared to Comparative Example, with statistical significance. The compositions of the present invention (G3 to G6) were found to have therapeutic effects equivalent to or higher than those of the conventional agent (G7).

### Experimental Example 2-3: Pathological Observation by H&E Staining

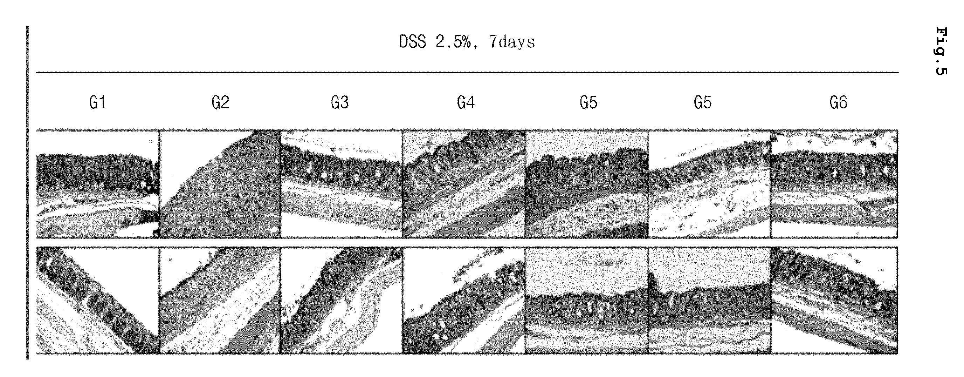

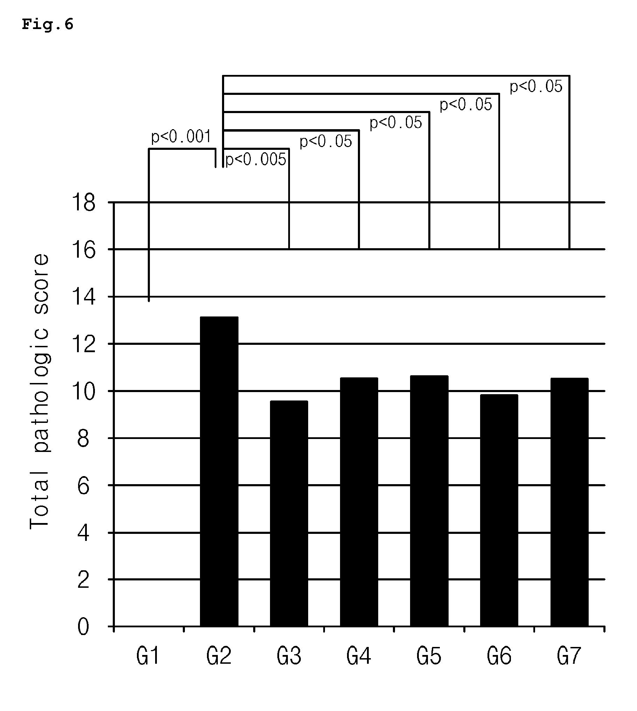

Colon specimens taken were fixed in a 10 % formalin solution and stained with hematoxylin & eosin (H&E stain), and their images are given in FIG. 5. Further, they were evaluated through a blind test under the criteria of a total of six histopathological opinions on inflammation (inflammation, follicle aggregation, oedema, erosion/ulceration, crypt loss, and infiltration of mono- and polymorphonuclear cells), and scored. Total pathologic scores obtained by summing up scores for each items and for the six opinions are depicted in FIG. 6.

As apparent from the data of FIGS. 5 and 6, the H&E staining assays of colon mucosal tissues show that intestinal mucosa tissues in which inflammation induced by DSS underwent a histopathological change due to inclammation, follicle aggregation, and oedema, and were deviated from the normal histological pattern. The treatment of G3 to G6 recovered the intestinal mucosa tissues to a normal state. Based on these results, histopathological evaluations were made, and depicted, indicating that higher pathologic scores were obtained in DSS-treated groups than the normal group while the treatment of G3 to G6 lowered the pathologic score. The composition of the present invention was found to have a therapeutic effect equivalent to or higher than that of the control G7.

### Experimental Example 2-4 : Level of Inflammatory Cytokine

Levels of IL-1β IL-6, IL-12, and TNF-α, which are representative inflammation-inducing cytokines, were measured. DSS-induced IBD groups were discovered to significantly increase in the expression of the inflammatory cytokines, and the expression level was significantly reduced by treatment with the composition of the present invention as in G3 to G6. The observation results are depicted in FIG. 5.

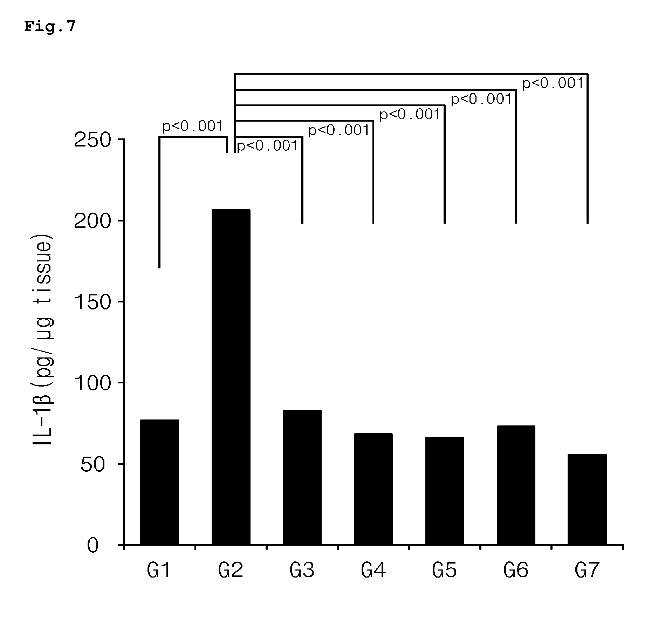

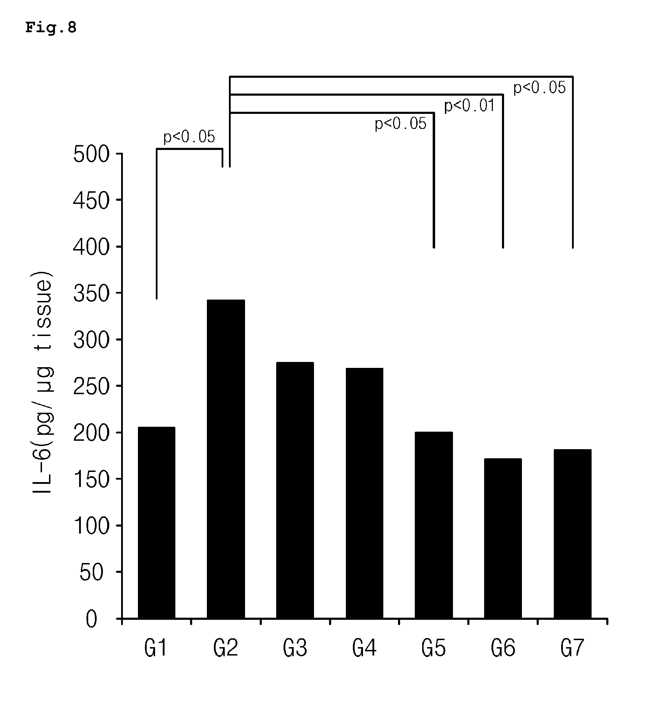

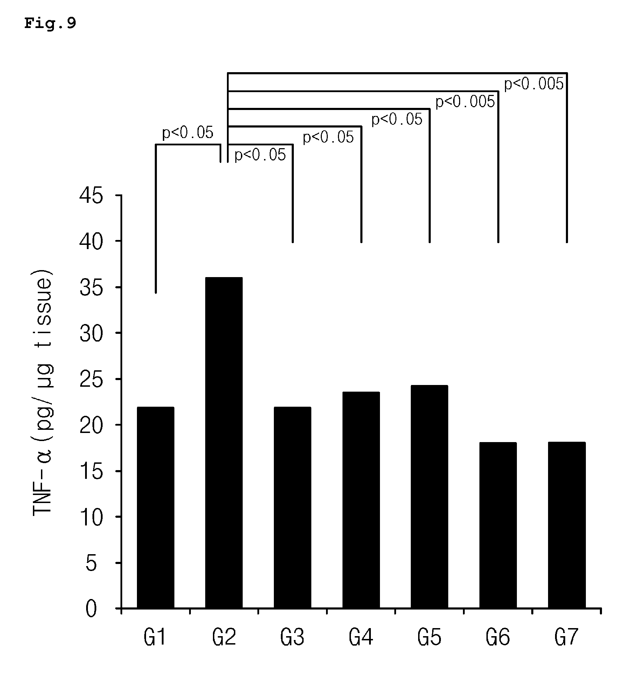

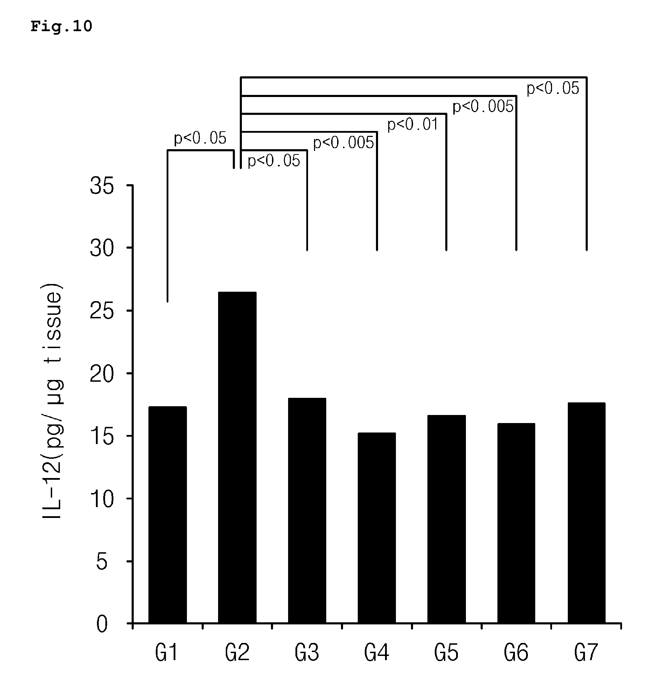

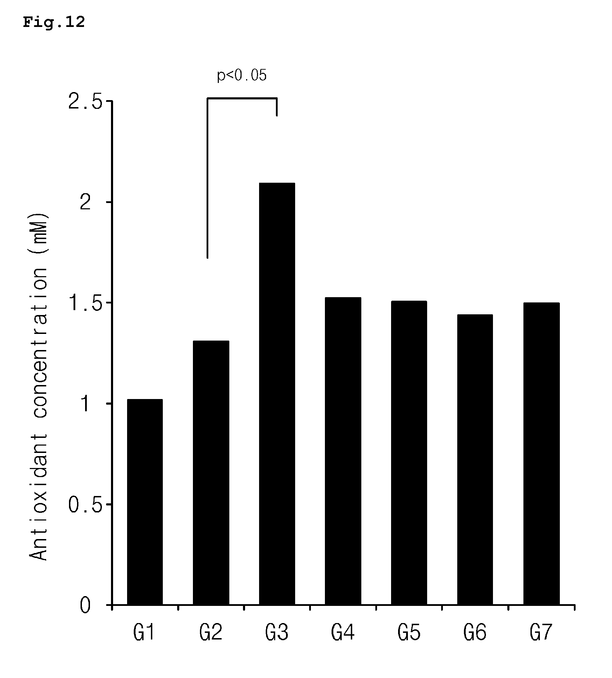

Data of FIGS. 7 and 10 show that DSS-induced inflammation promoted the expression of the inflammatory cytokines IL-1β (FIG. 7), IL-6 (FIG. 8), TNF-α (FIG.9), and IL-12 (FIG. 12), losing the function of the intestinal mucosa membrane, as demonstrated by H&E staining assay of FIG. 4, which resulted in a lesion phenomenon such as diarrhea and hematochezia. Treatment with the composition of the present invention as in G3 to G6 decreased expression levels of the inflammatory cytokines, which, in turn, rehabilitated the injured mucosal tissues to normal tissues, with equivalency to the control G7.

### Experimental Example 2-5: Assay for Anti-Oxidant Activity

Malondialdehyde (MDA), accounting for the degree of lipid peroxidation, a representative marker of oxidation stress, was quantified.

Its amount was discovered to decrease at a low concentration of S-allyl-L-cysteine as in G3 and G4, with statistical significance. Consistent with this result, an increase in antioxidant concentration was detected in G3, compared to the DSS-treated group, as measured for total antioxidant concentrations in tissues. These results are shown in FIGS. 10 (MDA concentration) and 11 (total antioxidant concentration).

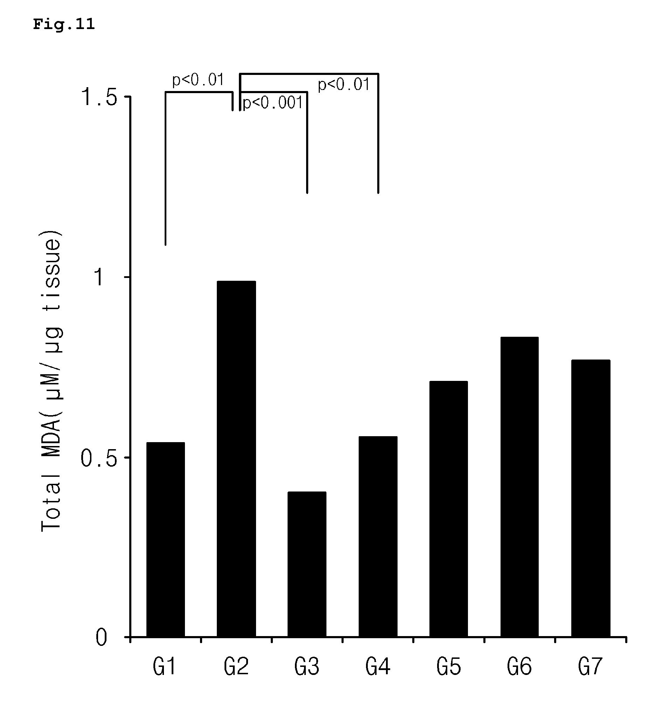

As is understood from the data of FIGS. 10 and 11, DSS-induced oxidative stress increased the level of MDA (malodialdehyde), a marker for lipid peroxidation, compared to the normal group. The increased level of lipid peroxidation induced inflammation in intestinal mucosal membranes, as confirmed by the H&E staining assay of FIG. 5, resulting in a functional loss of the mucosal membrane. Treatment of G3 to G6 decreased the level of MDA, accounting for a reduction in oxidative stress-induced inflammation. As such, the affected tissues were rehabilitated to normal mucosal tissues, with equivalency or superiority to the control G7. Particularly, the therapeutic effect peaked in the G3 group, which seemed to result from the highest intracellular antioxidant level. In addition, as evaluated by the total pathologic score of FIG. 6, the G3 group showed the best rehabilitation of mucosal tissues.

### Experimental Example 2-6: Expression Levels of Inflammatory Gene

COX-2, a representative inflammatory enzyme, significantly increased in expression level in the group G2 and G3. The expression level was observed to significantly decrease in the other test groups. In spite of many rounds thereof, Western blotting failed to obtain apparent results. The immunofluorescence of COX-2 was performed on paraffin unstained slides of animal tissues.

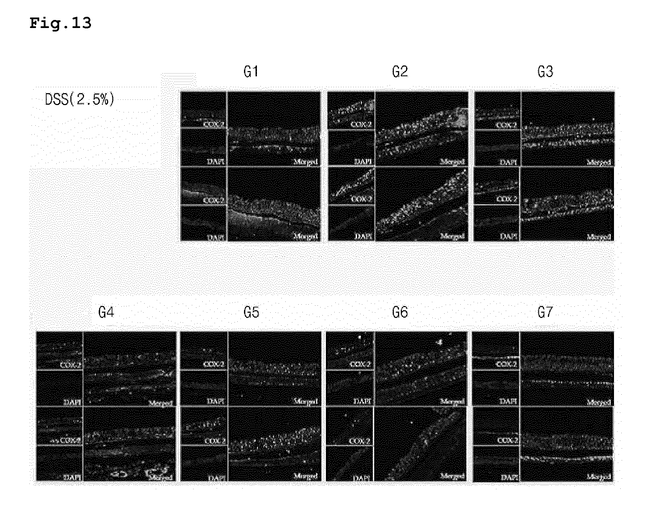

In all of the groups, the DSS-induced COX-2 expression was found to be suppressed. These results are shown in FIG. 13(COX-2 (green), DAPI (blue), and merged image).

As can be seen in FIG. 13, the expression level of COX-2, a representative marker of inflammation, was significantly increased in mucosal tissues of the colon where inflammation was elicited by DSS-induced oxidative stress, compared to the normal group. The COX-2 expression induced inflammation, as demonstrated by the H&E staining assay of FIG. 4, resulting in a functional loss of the mucosal membrane. Treatment of G3 to G6 reduced COX-2 expression levels in mucosal tissues, which, in turn, alleviated the oxidative stress-induced inflammation, thus rehabilitating the mucosal tissues to a normal state, with an equivalent or superior effect to that of the control G7. The control G7 inhibited COX-2 expression in mucosal tissues, but did not effectively do so in the fascial tissue beneath the mucosal tissue, whereas G4 to G6 inhibited COX-2 expression even in the fascial tissue.

### Experimental Example 2-7. Side Effects of S-Allyl-L-cysteine

Cyclosporine or prednisolone, which are both steroids used to treat colitis thanks to their immunosuppressive effects, are reported to have the side effect of weight gain. However, none of the mice in the test groups G3 to G6, which were administered with the composition of Example 1 containing S-allyl-L-cystein as an active ingredient, were found to gain weight. Thus, the composition of the present invention is proven to not cause the side effect of weight gain.

The anti-inflammatory agents including mesalizine or mesalazine, NSAIDs, etc. are known to cause various side effects such as hypersensitive reactions, nausea, vomiting, headache, etc. Over an extended test period of 12 weeks, G3 to G6, which were treated with S-allyl-L-cysteine, did not suffer from the side effects detected in the control G7 treated with mesalazine.

### EXAMPLES

### [FORMULATION EXAMPLES: Formulations Cotaining S-allyl-L-cysteine]

### Formulation Example 1: Preparation of Tablet

S-Allyl-L-cysteine: 200 mg
Lactose: 50 mg
Starch: 10 mg

### Formulation Example 2: Preparation of Powder

S-Allyl-L-cysteine: 250 mg
Lactose: 30 mg
Starch: 20 mg
Mg stearate: a suitable amount

These ingredients were well mixed, loaded to a polyethylene-coated sac, and sealed to give powders.

### Formulation Example 3: Preparation of Capsule

S-Allyl-L-cysteine: 500 mg
Lactose: 30 mg
Starch: 28 mg
Talc: 2 mg
Magnesium stearate suitable amount

The ingredients were mixed and loaded to gelatin hard capsules using a typical procedure to afford capsules.

### Formulation Example 4: Preparation of Suspension

S-Allyl-L-cysteine: 50 mg
Isomerized sugar: 10 g
Sugar: 30 mg
Sodium Carboxymethyl cellulose: 100 mg
Lemon aromatic: suitable amount
Pure water: added to form a total of 100 mL

These ingredients were mixed and formulated into a suspension using a typical method. The suspension was loaded to a 100 mL brown bottle, and sterilized.

### Formulation Example 5: Preparation of Soft Capsule (Content per capsule)

S-Allyl-L-cysteine: 500 mg
Polyethylene glycol: 400 mg
Conc. Glycerin: 55 mg
Pure Water: 35 mg

Polyethylene glycol and conc. Glycerin were mixed together, and then pure water was added. This mixture was maintained at about 60°C and homogeneously mixed with flavones by stirring at about 1,500 rpm in a stirrer. While slowly stirring, the resulting homogeneous mixture was cooled to room temperature, and degassed using a vacuum pump to give a soft capsule formulation. Using a typical method, a soft capsule shell was prepared from 132 mg of gelatin, 52 mg of conc. glycerin, 6 mg of 70 % disorbitol solution, and a suitable amount of ethyl vanillin as an aromatic, while carnauba wax was used as a coating base.

### Formulation Example 6: Preparation of Injection

S-allyl-L-cysteine 200 mg
Mannitol 180 mg
Sterile Distilled Water for injection 2974 mg
Na₂HPO₄12H₂O 26 mg

These ingredients were employed in the amounts per ampoule to give an injection using a typical method.

### Formulation Example 7: Preparation of Beverage

S-allyl-L-cysteine 0.01g
Citric acid: 8.5g
White sugar 10g
Glucose 2.5g
DL-malic acid 0.3g
Pure water: suitable amount

Pure water was added to these ingredients to form a total of 100 mL, and the solution was mixed to give a beverage using a typical method.

Although the preferred embodiment(s) of the present invention have been disclosed for illustrative purposes, those skilled in the art will appreciate that various modifications, additions, and substitutions are possible, without departing from the scope and spirit of the invention as disclosed in the accompanying claims.

As described hitherto, the composition comprising S-allyl-L-cysteine as an active ingredient is useful in preventing or treating colitis, and the use of the composition finds applications in various industries.

## Claims

1. A composition for preventing or treating colitis, comprising S-allyl-L-cysteine, a pharmaceutically acceptable salt thereof, or a solvate or hydrate thereof as an active ingredient.

2. The composition of claim 1, wherein S-allyl-L-cysteine is obtained by isolation and purification from the plant Allium genus, by synthesis, or by fermentation.

3. The composition of claim 1, further comprising an anti-inflammatory agent or an anti-oxidant agent.

4. The composition of claim 3, wherein the anti-inflammatory agent is selected from the group consisting of ibuprofen, ketoprofen, flurbiprofen, feno-profen, naproxen, piroxicam, tenoxicam, isoxicam, melo-xicam, indomethacin, aceclofenac, diclofenac, and a combination thereof, and the anti-oxidant agent is selected from the group consisting of vitamin A, vitamin C, vitamin E, carotenoid, zinc, copper, iron, manganese, lutein, zeaxanthin, selenium, glutathione (GSH), lycopene, and a combination thereof.

5. A medicinal formulation, comprising the composition of any one of claims 1 to 4, the medicinal formulation being selected from the group consisting of an oral dosage form, a mucosal application, an injection, an inhaler, and an external application.
